# EUROPÄISCHE PATENTSCHRIFT

(11) **EP 0 822 192 B1**
(45) Veröffentlichungstag und Bekanntmachung des Hinweises auf die Patenterteilung: **17.10.2001**
(21) Anmeldenummer: 97112940.8
(22) Anmeldetag: 28.07.1997
(51) Int. Cl.: C07D 309/30, C07D 335/02, C07D 211/78, C07F 9/655, C07D 211/74, A61K 31/35

(54) **Neue Inhibitoren der Beta-Glucuronidase**
Inhibitors of beta-glucuronidase
Inhibiteurs de bêta-glucuronidase

(30) Priorität: 02.08.1996 DE 19631288
(43) Veröffentlichungstag der Anmeldung: 04.02.1998
(73) Patentinhaber: HOECHST AKTIENGESELLSCHAFT, 65929 Frankfurt am Main (DE)
(72) Erfinder: Bosslet, Klaus Dr., Gaithersburg, MD 20879 (US); Czech, Jörg, Dr., 35041 Marburg (DE); Vasella, Andrea, 8057 Zürich (CH); Hoos, Roland Department of Organic Chemistry, Rehovot 76100 (IL)

(56) Entgegenhaltungen:
- US-A- 3 903 289
- R.HOOS ET AL.: "148.SYNTHESIS AND ENZYMATIC EVALUATION OF SUBSTRATES AND INHIBITORS OF BETA-GLUCURONIDASES." HELVETICA CHIMICA ACTA., Bd. 79, Nr. 7, 30.Oktober 1996, BASEL CH, Seiten 1757-1783, XP002053220

## Beschreibung

Diese Erfindung betrifft die Herstellung neuer Inhibitoren des Enzyms β-Glucuronidase sowie deren Anwendung als Inhibitoren des Tumorwachstums und der Metastasierung.

β-Glucuronidasen spielen eine Rolle beim Abbau des Polysaccharidanteils (Glycosaminoglycane) von Proteoglycanen, die ein Hauptbestandteil der extrazellulären Matrix sowie der endothelialen Basalmembran darstellen.
Glycosaminoglycane (Chondroitinsulfat, Dermatansulfat, Heparansulfat) enthalten β-Glucuronsäure als Bestandteil, an dem β-Glucuronidasen exo- und endoglycolytisch angreifen können.
Für Tumorzellen konnte gezeigt werden, daß ihr metastatisches Potential mit ihrer Heparanaseaktivität (Endo-β-glucuronidase) korreliert ist (M. Nakajima et al., Journal of Cellular Biochemistry 36:157-167, 1988). Weiter ist bekannt, daß Inhibitoren der Heparanase (M. Nakajima et al., Journal of Cellular Biochemistry 36:157-167, 1988) bzw. (Exo-)β-Glucuronidase (T. Niwa et al., Journal of Biochemistry 72: 207-211, 1972) in Modellsystemen die Metastasierung von Tumoren unterdrücken konnten.
im US-Patent 3,903,289 werden gesättigte heterocyclische Verbindungen beschrieben, die durch 3-N-Alkyl-carbamyloximino substituiert und als Pestizide einsetzbar sind.

Trotz dieser Befunde steht bis heute in der Klinik kein Inhibitor der β-Glucuronidase zur Unterdrückung der Tumormetastasierung zur Verfügung.

Aufgabe der vorliegenden Erfindung ist es neue Inhibitoren der β-Glucuronidase zu finden, die die Tumormetastasierung unterdrücken. Es wurden nun Verbindungen der Formel I, 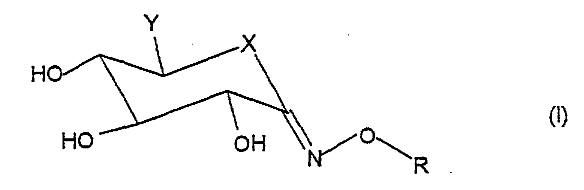 gefunden, die das obengenannte Kriterium erfüllen und dadurch gekennzeichnet sind, daß
- Y: für -COOH, -PO₃H₂, -P(O)(OR⁶)(OH), -P(O)R⁶(OH), Tetrazol oder -SO₃H steht, worin
R⁶ (C₁-C₄)-Alkyl bedeutet,
- X: für NH, O oder S steht,
- R: für Wasserstoffatom oder -C(O)NHC₆(R⁷)₅ steht, worin
R⁷ unabhängig voneinander Wasserstoffatom, OH, Halogen, -COOH, -PO₃H₂ oder -SO₃H bedeutet.

Eingeschlossen sind physiologisch verträgliche Salze der Verbindung der Formel I.

Bevorzugt sind Verbindungen der Formel II: 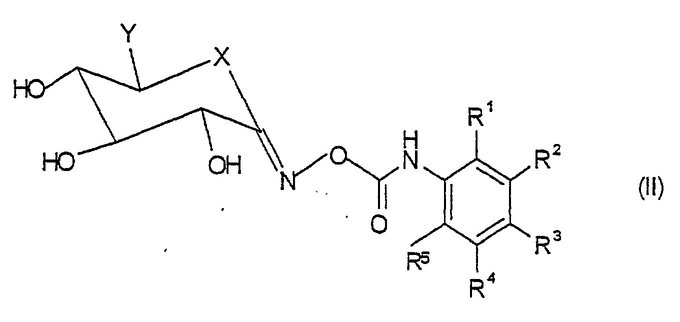 wobei
- Y: für -COOH, -PO₃H₂, -P(O)(OR⁶)(OH) oder -P(O)R⁶(OH) steht, worin
R⁶ (C₁-C₄)-Alkyl bedeutet,
- X: für NH oder O steht und
- R¹, R², R³, R⁴ oder R⁵: unabhängig voneinander für Wasserstoffatom, OH, Halogen, -COOH, -PO₃H₂ oder -SO₃H stehen.

Bevorzugte Salze der Verbindung der Formel II sind die Natriumsalze.

Besonders bevorzugt ist eine Verbindung der Formel II, wobei
- Y: für -COOH oder -PO₃H₂ steht,
- X: für NH oder O steht und
- R¹, R², R³, R⁴ oder R⁵: unabhängig voneinander für Wasserstoffatom oder Chlor stehen.

Die Verbindungen der Formel I oder II lassen sich nach bekannten Verfahren herstellen wie sie beispielsweise in EP 0 642 799 beschrieben werden.

Die Erfindung betrifft auch Arzneimittel, enthaltend mindestens eine Verbindung der . Formel I oder II und /oder ein physiologisch verträgliches Salz der Verbindung der Formel I und/oder II. Aufgrund der pharmakologischen Eigenschaften der Verbindungen der Formel I oder II können diese Verbindungen zur Behandlung von Krebserkrankungen oder der Unterdrückung der Tumormetastasierung, z.B. nach Operationen, eingesetzt werden. Ferner können entzündliche Erkrankungen behandelt werden.

Die Erfindung betrifft auch ein Verfahren zur Herstellung eines Arzneimittels, das dadurch gekennzeichnet ist, daß man die Verbindung der Formel I oder II und /oder ein physiologisch verträgliches Salz der Verbindung der Formel I und/oder II mit einem pharmazeutisch geeigneten und physiologisch annehmbaren Träger und gegebenenfalls weiteren geeigneten Wirk-, Zusatz- oder Hilfsstoffen in eine geeignete Darreichungsform bringt. Die erfindungsgemäßen Arzneimittel können oral, topisch, rektal, intravenös oder auch parenteral appliziert werden.

Geeignete feste oder flüssige galenische Darreichungsformen sind beispielsweise Granulate, Pulver, Dragees, Tabletten, (Mikro)Kapseln, Suppositorien, Sirupe, Säfte, Suspensionen, Emulsionen, Tropfen oder injizierbare Lösungen sowie Präparate mit protrahierter Wirkstoff-Freigabe, bei deren Herstellung übliche Hilfsmittel, wie Trägerstoffe, Spreng-, Binde-, Überzugs-, Quellungs-, Gleit- oder Schmiermittel, Geschmacksstoffe, Süßungsmittel oder Lösungsvermittler, Verwendung finden. Als häufig verwendete Hilfsstoffe seien z.B. Magnesiumcarbonat, Titandioxid, Laktose, Mannit und andere Zucker, Talkum, Milcheiweiß, Gelatine, Stärke, Cellulose und ihre Derivate, tierische und pflanzliche Öle, Polyethylenglycole und Lösungsmittel, wie etwas steriles Wasser und ein- oder mehrwertige Alkohole, z.B. Glycerin, genannt.

Vorzugsweise werden die pharmazeutischen Präparate in Dosierungseinheiten hergestellt und verabreicht, wobei jede Einheit als aktiven Bestandteil eine bestimmte Dosis der Verbindung der Formel I oder II und /oder physiologisch verträgliche Salze dieser Verbindungen enthält. Bei festen Dosierungseinheiten, wie Tabletten, Kapseln oder Suppositorien, kann diese Dosis bis zu etwa 1 g betragen.

Für die Behandlung eines erwachsenen Patienten (70 kg), sind in frühen Phasen eine intravenöse Infusionsbehandlung von maximal 4 g pro Tag und in der späteren Phase eine orale Verabreichung von 3 mal 1 g pro Tag der Verbindung der Formel I oder II und /oder einem physiologisch verträglichem Salz dieser Verbindung indiziert.

Unter Umständen können jedoch auch höhere oder niedrigere Dosen angebracht sein. Die Verabreichung der Dosis kann sowohl durch Einmalgabe in Form einer einzelnen Dosierungseinheit oder aber mehreren kleineren Dosierungseinheiten als auch durch Mehrfachgabe unterteilter Dosen in bestimmten Intervallen erfolgen.

Schließlich können die Verbindungen der Formel I oder II und /oder deren entsprechende Salze bei der Herstellung der vorgenannten galenischen Darreichngsformen auch zusammen mit anderen Wirkstoffen, beispielsweise Zytostatika, Proteaseinhibitoren, Neoangiogeneseinhibitoren oder anderen Inhibitoren der Tumormetastasierung, kombiniert werden.

Inhibitoren der β-Glucuronidase sowie deren Synthese werden in den folgenden Beispielen beschrieben.

### Beispiel 1:

Synthese von (Z)-O-(D-Glucopyranuronosyliden)amino N-Phenylcarbamat (Natriumsalz) (10):

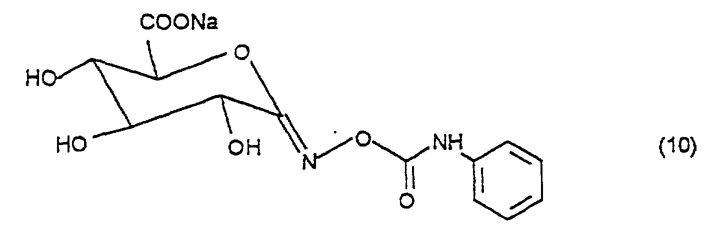

Benzyl (1,2,3,4-Tetra-O-acetyl-α/β-D-glucopyran)uronate (1/2).
Eine Lösung von Natriumglucuronat x H20 (2,00 g, 8,54 mmol) in DMF (30 ml) wurde mit BnBr (1,5 ml, 12,6 mmol) versetzt, bei ca. 23° für 24 h gerührt, durch Hyflo Super Cel® filtriert, und mit Ac₂O (20 ml) und Pyridin (40 ml) bei ca. 23° für 14 h behandelt. Die übliche Aufarbeitung (CHCl₃, waschen mit H₂O, H₂SO₄, und NaHCO₃ Lösung) und Flashchromathographie (FC) (an 80 g SiO₂) ergaben 1/2 1:1 (1,90 g, 49%). Eine Probe wurde aus Et₂O kristallisiert und ergab farblose Kristalle.
Schmelzpunkt 137,5 bis 139,5°.
R_{f} (Hexan/AcOEt 2:1) 0,17.
IR (CHCl₃): 2961w, 1761s, 1499w, 1456w, 1429w, 1369m, 1091m, 1041m, 913w.
¹H-NMR (300 MHz, CDCl₃): 1.77 (1.5 H), 1.80(1.5 H); 2.00 (3.0 H), 2.02 (1.5 H), 2.03(1.5 H), 2.09(1.5 H), 2.17(1.5 H, 4 AcO); 4.22 (d, J = 9.6, 0.5 H), 4.45 (d, J = 10.0, 0.5 H, H-C(5)); 5.09 bis 5.29 (m, 4.5 H); 5.49 (t, J = 9.9, 0.5 H); 5.77 (d, J = 7.6, 0.5 H), 6.40 (d, J = 3.7, 0.5 H, H-C(1)); 7.32 - 7.39 (m, 5 arom. H). ¹³C-NMR (75 MHz, CDCl₃): 20.25 - 20.82 (mehrere q); 68.01 (2t); 68.87 (2d); 69.06 (d); 69.15 (d); 70.20 (d); 70.49 (d); 71.94 (d); 73.01 (d); 88.79 (d); 91.35 (d); 128.35 - 128.84 (mehrere d); 134.55 (2s); 166.33 (s); 166.74 (s); 168.47 (s); 168.83 (s); 169,17 (s); 169.33 (2s); 169.52 (s); 169.89 (s), 170.00 (s). FAB-MS (3-NOBA): 475 (6, [M + Na]⁺), 394 (24), 393 (100, [M - OAc]⁺), 193 (48), 154 (22), 137 (57), 136 (29), 106 (25).

| | | |
|---|---|---|
| Analytische Berechnung für C₂₁ H₂₄O₁₁ (452,41) | C 55,75, | H 5,35; |
| gefunden | C 55,66, | H 5,41. |

Benzyl (2,3,4-Tri-O-acetyl-α/β-D-glucopyran)uronate (3/4).
Eine Lösung von 1/2 1:1 (6,35 g, 14.04 mmol) in DMF (100 ml) wurde mit (NH₄)₂CO₃ (3,18 g) bei ca. 22° für 4 h behandelt, auf 5° gekühlt, und mit CH₂Cl₂ (200 ml) und Eis (ca. 400 ml) versetzt. Extraktion mit CH₂Cl₂, waschen der vereinten organischen Phasen mit 0,5 M H₂SO₄ und gesättigter wäßrigen NaHCO₃ Lösung, trocknen (MgSO₄), eindampfen, und FC (Hexan/AcOEt 1:1) ergaben 3/4 4:1 (3,85 g, 67%) als farbloses Öl. R_{f} (Hexan/AcOEt 1:2) 0,44.
IR (CHCl₃): 3594w, 2960w, 1754s, 1498w, 1456w, 1429w, 1369m, 1146w, 1065m, 1040m, 908w.
¹H-NMR (200 MHz, CDCl₃) von 71/72, Signale von 3: 1.77, 2.01, 2.08 (3s, 3 AcO); 3.50 (br. s, ca. 1 H, ausgetauscht mit CD₃OD, OH); 4.62 (d, J = 10.1, H-C(5)); 4.92 (dd, J = 10.1, 3.6, H-C(2)); 5.10 (d, J = 12.0), 5.19 (d, J = 12.0, PhCH₂); 5.20 (t, J ≈ 10.1, H-C(4)); 5.55 (d, J ≈ 3.6, Zugabe von CD₃OD zu höheren Feldern von 0.13 ppm), H-C(1)); 5.56 (t, J ≈ 9.7, H-C(3)); 7.35 (s, 5 arom. H). ¹³C-NMR (75 MHz, CDCl₃) von 3/4 4:1, Signale von 3: 20.30 - 20.67 (mehrere q); 67.95 (t); 67.98 (d); 69.30 (d); 69.50 (d); 70.74 (d); 90.19 (d); 128.66- 128.86 (mehrere d); 134.59 (s); 168.13 (s); 169.69 (s); 170.12 (s); 170.24 (s); signals of 72: 20.30- 20.67 (several q); 68.07 (t); 69.34 (d); 71.76 (d); 72.54 (d); 72.75 (d); 95.45 (d); 128.66- 128.86 (mehrere d); 134.52 (s); 167.17 - 170.45 (mehrere s). FAB-MS (3-NOBA): 433 (11, [M + Na]⁺), 394 (17), 393 (76, [M - OH]⁺), 307 (19), 303 (13), 289 (14), 193 (29), 155 (38), 154 (100).

Benzyl (E/Z)-2,3,4-Tri-O-acetyl-D-glucuronate 1-Oxime (5/6).
Eine Lösung von 3/4 4:1 (3,85 g, 9.38 mmol) in Pyridin (55 ml) wurde bei ca. 23° für 2,5 h mit H₂NOH·HCl (1,96 g, 28,2 mmol) behandelt, mit CH₂Cl₂/H₂O verdünnt, und geschüttelt. Das Waschen der organischen Phase mit 0,5 M H₂SO₄ und gesättigter wäßrigen NaHCO₃ Lösung, trocknen (MgSO₄), und eindampfen ergab 5/6 7:3 (3,45 g, 86%) als gelben Schaum, der so weiter verwendet wurde.
Rf (Hexan/AcOEt 1:2) 0,35 (5), 0,29 (6).
IR (CHCl₃): 3573w, 3038w, 1751s, 1498w, 1456w, 1428w, 1373m, 1086m, 1045m, 958w.
¹H-NMR (500 MHz, CDCl₃) von 5/6 7:3: 1.85, 1.86, 2.05, 2.09, 2.09, 2.10 (6s, 3 AcO); 3.43 (br. s, ausgetauscht mit CD₃OD, HO-C(5)); 4.21 (d, J = 7.4, 0.7 H), 4.23 (d, J = 6.3, 0.3 H, H-C(5)); 5.11 (d, J = 11.9, 0.7 H), 5.20 (d, J ≈ 12.1, 0.7 H); 5.14 (d, J = 12.0, 0.3 H), 5.22 (d, J ≈ 12.7, 0.3 H, PhCH₂); 5.22 (dd, J ≈ 6.3, 5.1, 0.3 H), 5.23 (dd, J= 7.4, 3.5, 0.7 H, H-C(4)); 5.56 (dd, J = 7.1, 5.8, 0.7 H), 6.14 (t, J = 5.7, 0.3 H, H-C(2)); 5.67 (dd, J = 7.1, 3.5, 0.7 H), 5.76 (dd, J = 5.6, 4.7, 0.3 H, H-C(3)); 6.55 (d, J = 5.8, 0.3 H), 7.29 (d, J = 5.8, 0.7 H, H-C(1)); 7.33-7.40 (m, 5 arom. H); 8.34 (br. s, ausgetauscht mit CD₃OD, 0.7 H), 8.55 (br. s, ausgetauscht mit CD₃OD, 0.3 H, NOH).
¹³C-NMR (125 MHz, CDCl₃) von 5/6 7:3, Signale von 5: 20.47 - 20.64 (mehrere q); 68.55 (t); 68.95 (d); 69.34 (d); 69.81 (d); 71.11 (d); 128.68- 129.05 (mehrere d); 134.37 (s); 145.50 (d); 169.60 (s); 169.81 (s); 170.20 (s); 171.81 (s); Signale von 6: 65.65 (d); 68.55 (t); 69.25 (d); 69.42 (d); 71.79 (d); 134.46 (s); 146.41 (d); 169.63 (s); 169.85 (s); 170.34 (s); 171.63 (s). FAB-MS (3-NOBA): 427 (26), 426 (100, [M + 1]⁺), 366 (22).

(Z)-2,3,4-Tri-O-acetyl-D-glucanhydroximo-1,5-lacton Benzyl Ester (7).
Eine Lösung von 5/6 7:3 (3,15 g, ca. 7,41 mmol) in CH₂Cl₂ (100 ml) wurde mit DBU (1,36 g, 8,93 mmol) und NCS (1,19 g, 8,91 mmol) bei -78° behandelt, und innerhalb von 30 Minuten auf ca. 23° erwärmt. Es wurde mit CH₂Cl₂ und H₂O versetzt, geschüttelt, die Phasen wurden getrennt, anschließend wurde die organische Phase getrocknet (MgSO₄) und einer FC unterworfen (an 100 g SiO₂, Hexan/AcOEt 2:1). Es wurde 7 (2,40 g, 77% von 3/4) als hygroskopischen Schaum erhalten.
R_{f} (Hexan/AcOEt 1:2) 0,33.
IR (CHCl₃): 3573w, 1760s, 1680w, 1498w, 1456w, 1372m, 1145w, 1097w, 1053m, 963w, 909w.
¹H-NMR (300 MHz, CDCl₃): 1.91, 2.03, 2.13 (3s, 3 AcO); 4.97 (d, J = 5.5, H-C(5)); 5.18 (t, J ≈ 3.7, H-C(3)); 5.22 (d, J = 12.1), 5.28 (d, J ≈ 12.7, PhCH2); 5.30 (s, Zugabe von CD₃OD - teilweiser Austausch, OH); 5.36 (dd, J ≈ 5.0, 4.2, H-C(4)); 5.49 (d, J = 3.9, H-C(2)); 7.36- 7.40 (m, 5 arom. H). ¹³C-NMR (75 MHz, C₆D₆): 19.87 (q); 20.03 (q); 20.16 (q); 66.73 (d); 68.04 (t); 69.70 (d); 70.69 (d); 75.23 (d); 128.75 (d); 128.87 (2d); 128.94 (2d); 135.34 (s); 147.53 (s); 166.76 (s); 168.67 (s); 168.97 (s); 169.05 (s). FAB-MS (3-NOBA): 426 (6), 425 (25), 424 (100, [M + 1]⁺), 307 (16).

| | | | |
|---|---|---|---|
| Analytische Berechnung für C₁₉H₂₁O₁₀ (423, 37) | C 53,90, | H 5,00, | N 3,31; |
| gefunden | C 53,37, | H 5,40, | N 3,39. |

O-((Z)-2,3,4-Tri-O-acetyl-D-glucopyranuronosyliden)amino Benzyl Ester N-Phenylcarbamat (8).
Eine Lösung von 7 (500 mg, 1,18 mmol) in CH₂Cl₂ (20 ml) wurde mit PhNCO (0,25 ml, 2,29 mmol) und iPr₂EtN (30 ml, 0,18 mmol) bei 0° behandelt, sofort auf 23° erwärmt, und 30 min gerührt. Nach Eindampfen und FC ergaben sich 8 (492 mg, 77%) als Schaum.
R_{f} (Hexan/AcOEt 1:1) 0,36. [α]= 15,4 (c= 1,14, CHCl₃).
IR (CHCl₃): 3393w, 3038w, 1762s, 1670w, 1602w, 1523m, 1445m, 1372m, 1312w, 1178m, 1133w, 1101m, 1052m, 1010m.
¹H-NMR (300 MHz, CDCl₃): 1.98, 2.03, 2.18 (3s, 3 AcO); 5.03 (d, J = 5.6, H-C(5)); 5.21 (t, J ≈ 3.5, H-C(3)); 5.23 (d, J ≈ 13.2), 5.28 (d, J = 11.9, PhCH₂); 5.37 (dd, J ≈ 5.6, 3.1, H-(4)); 5.64 (d, J = 4.0, H-C(2)); 7.09 - 7.48 (m, 10 arom. H); 7.79 (br. s, NH).
¹³C-NMR (75 MHz, CDCl₃): 20.43 (q); 20.50 (q); 20.66 (q); 65.54 (d); 68.50 (t); 68.83 (d); 69.64 (d); 75.42 (d); 119.31 (2d); 124.22 (d); 128.76 (2d); 128.82 (2d); 128.99 (d); 129.14 (2d); 134.18 (s); 136.92 (s); 150.57 (s); 150.94 (s); 165.80 (s); 168.26 (s); 168.59 (s); 168.91 (s). FAB-MS (3-NOBA): 545 (7), 544 (31), 543 (100, [M + 1]⁺), 424 (22), 423 (13), 307 (30), 289 (17).

| | | | |
|---|---|---|---|
| Analytische Berechnung für C₂₆H₂₆O₁₁ (542,50) | C 57,56, | H 4,83, | N 5,16; |
| gefunden | C 57,58, | H 5,01, | N 5,29. |

(Z)-O-(D(Z)-O-(D-Glucopyranuronosyliden)amino )amino N-Phenylcarbamat (Natriumsalz)(10).
Eine Lösung von 8 (255 mg, 0,47 mmol) in MeOH (6 ml) wurde mit H₂ (1-2 bar) in der Gegenwart von Pd/C (10%, 5 mg) bei ca. 23° 30 min lang behandelt. Als TLC das Ende der Reaktion anzeigte (neuer Fleck bei R_{f} (AcOEt/MeOH/H₂O 7:2:1) 0,32), wurde eine Lösung von NH₃ in MeOH (3,0 ml) tropfenweise hinzugegeben. Nach 3 h wurde die Mischung durch Hyflo Super Cel® filtriert und eingedampft. Der Rückstand, 9, wurde in H₂O gelöst, und durch eine mit Dowex® 50W X2 (50 - 100 mesh, Na⁺ Form) gepackte Säule filtriert. Die (Z)-O-(D-Glucopyranuronosyliden)amino N-Phenylcarbamat enthaltenden Fraktionen wurden gesammelt und auf eine mit LiChroprep® RP-18 (40-63 mm) gepackte Säule gegeben. Elution mit H₂O, Lyophilisation, Ausfällung aus MeOH mit EtOH, und Lyophilisation ergaben das Natriumsalz von (Z)-O-(D-Glucopyranuronosyliden)amino N-Phenylcarbamat (10).
R_{f} 0,47.
IR (KBr) von 10: 3380s, 1751m, 1620s, 1550m, 1501w, 1447m, 1406w, 1318w, 1254w, 1211m, 1110w, 1062w, 1020m, 753w.
¹H-NMR (200 MHz, CD₃OD) von 9: 3.73 (dd, J = 7.0, 4.9, H-C(4)); 4.02 (t, J ≈ 5.5, H-C(3)); 4.40 (d, J = 7.2, H-C(5)); 4.56 (d, J = 6.1, H-C(2)); 7.01 - 7.09 (m, 1 arom. H); 7.25 - 7.33 (m, 2 arom. H); 7.42- 7.52 (m, 2 arom. H). ¹³C-NMR (50 MHz, CD₃OD) von 9: 70.73 (d); 75.07 (d); 77.66 (d); 82.21 (d); 120.60 (d); 124.92 (2d); 130.21 (2d); 139.79 (s); 155.52 (s); 161.00 (s); 174.92 (s). FAB(-)-MS (Glycerin) von 10: 325 (32, [M - Na⁺]⁻), 183 (100), 181 (41).

### Beispiel 2:

### Synthese von Natrium (5R, Z)-O-(5-C-Phosphonato-D-xylopyranosyliden)amino N-Phenylcarbamat (9)

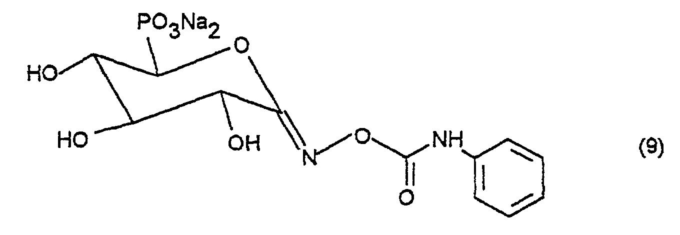

(5R)-1,2,3,4-Tetra-O-acetyl-5-C-(diphenyloxyphosphoryl)-α/β-D-xylopyranose (1/2). Eine Lösung von 3-O-Acetyl-1,2-O-isopropyliden-α-O-glucofuranose (60,0 g, 229 mmol) in H₂O (900 ml) wurde mit NaIO₄ (55,0 g, 257 mmol) behandelt, 15 min gerührt, und nochmals mit NaIO₄ (17,0 g, 79 mmol) behandelt. Nach 1 h, wurde die Lösung mit CHCl₃ (10 x 300 ml) extrahiert, und die vereinigten organischen Phasen wurden eingedampft. Der Rückstand wurde in CH₂Cl₂ (300 ml) gelöst, mit frisch destilliertem HOP(OPh)₂ (53 ml, 275 mmol) und iPr₂EtN (2 ml, 12 mmol) versetzt, 30 min gerührt, und eingedampft. Der Rückstand (110g) wurde in AcOEt/HCO₂H/H₂O 4:4:1(536 ml) gelöst, unter Rückfluß 90 min erhitzt, und eingedampft. Der Rückstand wurde in Toluol (800 ml) und 1,4-Dioxan (300 ml) suspendiert. H₂O und HCO₂H wurden in einem Dean-Stark Apparat bei ca. 80 mbar entfernt. Nach dem Eindampfen wurde der Rückstand in Ac₂O (120 ml) suspendiert und mit 70% HClO₄ (4 x 2 ml) unter heftigem Schütteln behandelt bis sich eine klare rötliche Lösung bildete. Die Lösung wurde auf Eis (ca. 600 ml) gegossen, mit Pyridin (10 ml) und CHCl₃ (250 ml) behandelt, und 60 Minuten heftig gerührt. Phasentrennung, Extraktion der wäßrigen Phase mit CHCl₃ (3 x 250 ml), waschen der vereinten organischen Phasen mit gesättigter wäßriger NaHCO₃-Lösung ( pH 11), trocknen (MgSO₄), eindampfen, und FC (an 800 g SiO₂, Hexan/AcOEt 1:1) ergaben 1/2 3:2 (¹H=NMR; 19,8 g, 16%).
Daten von 1: Farbloses Öl.
R_{f} (Hexan/AcOEt 1:1) 0,21. [α]= 98,2 (c = 0,62, CHCl₃).
IR (CHCl₃): 3008w, 2959w, 1758s, 1591m, 1490s, 1371m, 1161s, 1084m, 1047s, 1026m, 1010m, 986w, 957s, 986w, 957s, 906w, 838w.
FAB-MS (3-NOBA): 552 (10), 551 (33, [M + 1]⁺), 457 (21), 330 (31), 329 (100), 301 (12).

| | | |
|---|---|---|
| Analytische Berechnung für C₂₅H₂₇O₁₂P (550,45) | C 54,55, | H 4,94; |
| gefunden | C 54,55, | H 4,81. |

Daten von 2: Farblose Kristalle. Schmelzpunkt 107,0-108,0° C (Hexan/Et₂O).
R_{f} (Hexan/AcOEt 1:1) 0,24. [a]= 32,4 (c = 0,50, CHCl₃).
IR (CHCl₃): 3008w, 1762s, 1591m, 1490s, 1369m, 1273m, 1161m, 1073s, 1040s, 958s, 906m.
FAB-MS (3-NOBA): 551 (5, [M + 1]⁺), 492 (11), 491(40), 457(6), 389(12), 330 (26), 329 (100), 308(8), 307 (30), 289 (17), 253 (14).

| | | |
|---|---|---|
| Analytische Berechnung für C₂₅H₂₇O₁₂P (550,45) | C 54,55, | H 4,94; |
| gefunden | C 54,35, | H 4,83. |

(5R)-2,3,4-Tri-O-acetyl-5-C-(dibenzyloxyphosphoryl)-a/ß-D-xylopyranose (3/4). Eine Lösung von 1/2 3:2 (1 g, 3,30 mmol) in BnOH (3,7 ml) wurde mit Ti(OiPr)₄ (0,8 ml) bei 60° behandelt und ca. 90 min gerührt. Aufarbeitung und FC (Hexan/AcOEt 3:2 - 1:1 - 1:3) ergaben 3/4 7:1 (341 mg, 35%).
Daten von 3/4: Lange farblose Nadeln. Schmelzpunkt 100,5-102,0 °C
(CH₂Cl₂/Hexan).
R_{f} (Hexan/AcOEt 1:3) 0,25. [α]= 90,2 (ca. 10 min) 73,2 (24 h) (c = 1,30, CHCl₃).
IR (CHCl₃): 3550w, 3275w, 3069w, 3008m, 2962w, 1752s, 1603w, 1456m, 1369m, 1161m, 1040s, 1009s, 999s, 965m, 922w, 887w, 601m.
³¹P-NMR(121 MHz, CDCl₃) of 3/4 7:1,
Signale von 4: 17.45. FAB-MS (3-NOBA): 538(31), 537 (100, [M + 1]⁺), 391 (22), 307 (26), 155 (18), 154 (44), 138 (23), 137 (41), 136 (33), 123 (17), 91 (54).

| | | | |
|---|---|---|---|
| Analytische Berechnung für C₂₅H₂₉O₁₁P (536,47) | C 55,97, | H 5,45, | P 5,77 |
| gefunden | C 55,96, | H 5,63, | P 5,49. |

(5R, E/Z)-2,3,4-Tri-O-acetyl-5-C(dibenzyloxyphosphoryl)-D-xylose Oxim (5/6).
Eine Lösung von 3/4 (500 mg, 0,932 mmol) in Pyridin (12,5 ml) wurde mit H₂NOH·HCl (195 mg, 2,81 mmol) für 4 h bei ca. 23° behandelt, mit CH₂Cl₂ und
H₂O verdünnt, und geschüttelt. Das Waschen der organischen Schicht mit 0,5M H₂SO₄ und gesättigter wäßriger NaHCO₃-Lösung, trocknen (MgSO₄), und eindampfen ergab 5/6 (487 mg, 95%) als gelbes Öl, das sofort für den nächsten Schritt benutzt wurde.
R_{f} (Hexan/AcOEt 1:4) 0,24 (5), 0,19 (6).
IR (CHCl₃): 3574w, 3249m, 3093w, 3069w, 3008w, 2959w, 1751s, 1603w, 1498w, 1456m, 1431w, 1373s, 1041s, 998s, 967m, 869w, 601m. FAB-MS (3-NOBA): 552 (8, [M + 1]⁺), 189(8), 181 (9), 171 (23), 170 (100), 136 (29).

(5R, Z)-2,3,4-Tri-O-acetyl-5-C-(dibenzyloxyphosphoryl)-D-xylanhydroximo-1,5-lacton (7).
Eine Lösung von 5/6 (300 mg, ca. 0,54 mmol) in CH₂Cl₂ (12 ml) wurde mit einer Lösung von DBU (95 mg, 0,62 mmol) in CH₂Cl₂ (3 ml) und NCS (84 mg, 0,63 mmol) bei -78° behandelt, und in 30 Minuten auf ca. 23° erwärmt. Zugabe von CH₂Cl₂ und H₂O, schütteln, Phasentrennung, trocknen der organischen Phase (MgSO₄), und FC (an 30 g SiO₂, Hexan/AcOEt 1:1) ergab 7 (269 mg, 88% von 3/4) als farblosen Schaum.
R_{f} (Hexan/AcOEt 1:2) 0,28. [α]= +50,2 (c = 0,83, CHCl₃).
IR (CHCl₃): 3265w, 3008m, 1754s, 1498W, 1456m, 1430w, 1374s, 1044s, 998s, 967m, 871w.
³¹P-NMR (121 MHz, CDCl₃): 15.53.
FAB-MS (3-NOBA): 552 (9), 551 (37), 550 (100, [*M* + 1]⁺), 388 (10).

| | | | |
|---|---|---|---|
| Analytische Berechnung für C₂₅H₂₈NO₁₁P (549,47) | C 54,65, | H 5,14, | N 2,55; |
| gefunden | C 54,37, | H 5,35, | N 2,33. |

(5R,Z)-O-(2,3,4-Tri-O-acetyl-5-C-(dibenzyloxy-phosphoryl)-D-xylopyranosyliden)amino N-Phenylcarbamat (8).
Eine Lösung von 7 (182 mg, 0,33 mmol) in CH₂Cl₂ (6 ml) wurde bei 0° für 30 min mit PhNCO (72 ml, 0,66 mmol) und iPr₂EtN (30 ml, 0,18 mmol) behandelt.
Eindampfen und FC (an 50 g SiO₂, Hexan/AcOEt 1:1) ergab 8 (217 mg, 98%) als Schaum.
R_{f} (Hexan/AcOEt 1:2) 0,32. [α]= +53,2 (c = 0,75, CHCl₃).
IR (CHCl₃): 3393w, 3008w, 2964w, 1762s, 1669m, 1602m, 1522m, 1456w, 1445m, 1373m, 1311w, 1296w, 1043s, 1008s, 996s.
FAB-MS (3-NOBA): 670 (35), 669 (100, [M + 1]⁺), 551 (20), 550 (68, [M + 1 - PhNCO]⁺), 490 (25), 460(21), 307 (27), 182 (20), 181 (39).

| | | | |
|---|---|---|---|
| Analytische Berechnung für C₃₂H₃₃N₂O₁₂P (668,59) | C 57,49, | H 4,97, | N 4,19; |
| gefunden | C 57,20, | H 5,15, | N 4,21. |

Natrium (5R,Z)-O-(5-C-Phosphonato-D-xylopyranosyliden) amino N-Phenylcarbamat (9).
Eine Mischung von 8 (180 mg) und Pd/C (10%, 5 mg) in MeOH (6 ml) wurde mit H₂ bei 1 atm für 30 min behandelt und als TLC das Ende der Reaktion durch das Entstehen eines neuen Flecks bei R_{f} (AcOEt/MeOH/H₂O 4:2:1) 0,50 anzeigte, wurde tropfenweise eine Lösung von NH₃ in MeOH (4,5 ml) dazugegeben. Nach Ende der Reaktion (3 h) wurde die Mischung durch Hyflo Super Cel® filtriert, eingedampft, in H₂O gelöst, und durch eine mit Dowex® 50W X2 50-100 mesh (Na⁺ Form) gepackte Säule filtriert. Die 9 enthaltenden Fraktionen wurden gesammelt und auf eine Säule mit LiChroprep® RP-18 (40-63 mm) gegeben, die mit H₂O eluiert wurde. Lyophilisation, Ausfällen aus H₂O mit EtOH, und lyophilisieren ergab reines 9 (50 mg, 48%).
Rf (AcOEt/MeOH/H₂O 4:2:1) 0,23.
IR (KBr): 3407s, 1750m, 1654m, 1604m, 1558m, 1502w, 1447m, 1318w, 1256w, 1214m, 1082s, 976m, 907w.
³¹P-NMR (162 MHz, D₂O): -2.63. FAB(-)-MS (Glycerin): 361(16, [M - Na⁺]⁻), 275 (17), 183 (100),181 (38).

### Beispiel 3:

### Inhibition der β-Glucuronidase

Die Inhibition der humanen β-Glucuronidase wurde mit 4-Methylumbelliferyl-β-D-glucuronid als Substrat bestimmt. Der Testansatz enthielt 75 µl 2,5 mM 4-Methylumbelliferyl-ß-D-glucuronid, 25 µl Enzym (0,6 µg/ml) and 10 µl der Verbindung der Formel I in einem Konzentrationsbereich von 0,0001 bis 100 mM alles in 200 mM Natriumacetat-Puffer und 0,1 mg/ml Rinderserumalbumin (BSA), pH 5. Nach einer 10-minütiger Inkubation bei 37°C wurde der Ansatz mit 1,5 ml 0,2 M Glyzin / 0,2 % SDS, pH 11,7, abgestoppt. Das vom Enzym freigesetzte Methylumbelliferon wurde in einem Fluoreszenzphotometer bei 360 nm Anregungs- und 450 nm Emissionswellenlänge gemessen.

Aus der Inhibitorverdünnungsreihe wurden die IC₅₀-Werte mit dem Softwareprogramm GraFit 2,0 (Erithacus Software Ltd.) berechnet.

| | IC₅₀ (mM) |
|---|---|
| (Z)-O-(D-Glucopyranuronosyliden)amino N-Phenylcarbamat (Natriumsalz) (Beispiel 1) | 0,0006 |
| Natrium (5R,Z)-O-(5-C-Phosphonato-D-xylopyranosyliden)amino N-Phenylcarbamat (Beispiel 2) | 11 |

## Patentansprüche

1. Verbindung der Formel I, 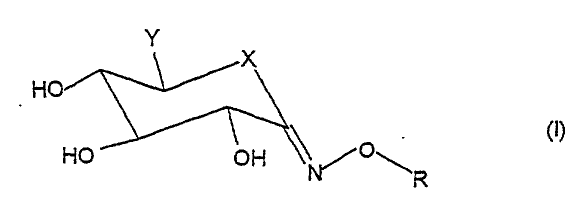 und/oder ein physiologisch verträgliches Salz der Verbindung der Formel I, wobei
Y für-COOH, -PO₃H₂, -P(O)(OR⁶)(OH), -P(O)R⁶(OH), Tetrazol oder -SO₃H steht, worin
R⁶ (C₁-C₄)-Alkyl bedeutet,
X für NH, O oder S steht,
R für Wasserstoffatom oder -C(O)NHC₆(R⁷)₅ steht, worin
R⁷ unabhängig voneinander Wasserstoffatom, OH, Halogen, -COOH, -PO₃H₂, oder -SO₃H bedeutet.

2. Verbindung der Formel II, gemäß Anspruch 1 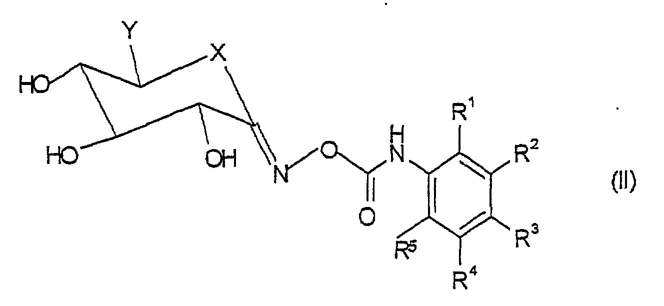 und/oder ein physiologisch verträgliches Natriumsalz der Verbindung der Formel II, wobei
Y für -COOH, -PO₃H₂, -P(O)(OR⁶)(OH) oder -P(O)R⁶(OH) steht, worin
R⁶ (C₁-C₄)-Alkyl bedeutet,
X für NH oder O steht und
R¹, R², R³, R⁴ oder R⁵ unabhängig voneinander für Wasserstoffatom, OH, Halogen, -COOH, -PO₃H₂ oder -SO₃H
stehen.

3. Verbindung der Formel II gemäß Anspruch 1 oder 2, wobei
Y für -COOH oder -PO₃H₂ steht,
X für NH oder O steht und
R¹, R², R³, R⁴ oder R⁵ unabhängig voneinander für Wasserstoffatom oder Chlor stehen.

4. Arzneimittel, enthaltend mindestens eine Verbindung der Formel I oder II gemäß einem oder mehreren der Ansprüche 1 bis 3.

5. Verwendung einer Verbindung der Formel I oder II gemäß einem oder mehreren der Ansprüche 1 bis 3 zur Herstellung eines Arzneimittels zur Behandlung von Krebserkrankungen.

6. Verwendung einer Verbindung der Formel I oder II gemäß einem oder mehreren der Ansprüche 1 bis 3 zur Herstellung eines Arzneimittels zur Prävention der Tumormetastasierung.

7. Verwendung einer Verbindung der Formel I oder II gemäß einem oder mehreren der Ansprüche 1 bis 3 zur Herstellung eines Arzneimittels zur Behandlung von entzündlichen Erkrankungen.

## Claims

1. A compound of the formula I 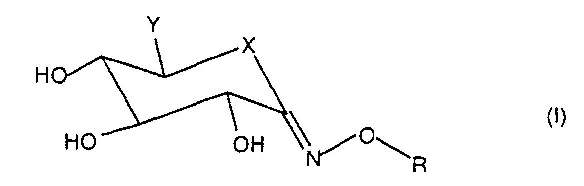 and/or a physiologically tolerable salt of the compound of the formula I, where
Y is -COOH, -PO₃H₂, -P(O) (OR⁶) (OH), -P(O)R⁶(OH), tetrazole or -SO₃H in which
R⁶ is (C₁-C₄)-alkyl,
X is NH, O or S,
R is a hydrogen atom or -C(O)NHC₆(R⁷)₅, in which
R⁷ independently of one another is a hydrogen atom, OH, halogen, -COOH, -PO₃H₂, or -SO₃H.

2. A compound of the formula II as claimed in claim 1 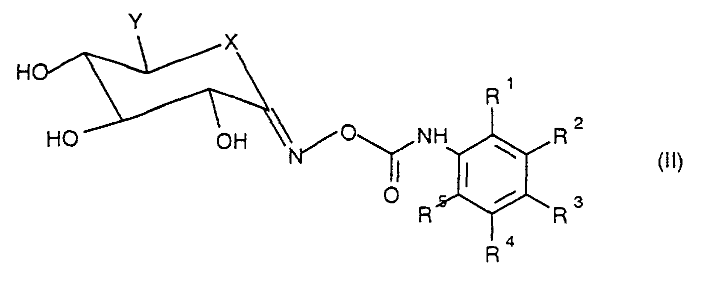 and/or a physiologically tolerable sodium salt of the compound of the formula II, where
Y is -COOH, -PO₃H₂, -P(O) (OR⁶) (OH) or -P(O)R⁶(OH), in which
R⁶ is (C₁-C₄)-alkyl,
X is NH or O and
R¹, R², R³, R⁴ or R⁵ independently of one another are a hydrogen atom, OH, halogen, -COOH, -PO₃H₂ or -SO₃H.

3. A compound of the formula II as claimed in claim 1 or 2, where
Y is -COOH or -PO₃H₂,
X is NH or O and
R¹, R², R³, R⁴ or R⁵ independently of one another are a hydrogen atom or chlorine.

4. A pharmaceutical comprising at least one compound of the formula I or II as claimed in one or more of claims 1 to 3.

5. The use of a compound of the formula I or II as claimed in one or more of claims 1 to 3 for the production of a pharmaceutical for the treatment of carcinomatous disorders.

6. The use of a compound of the formula I or II as claimed in one or more of claims 1 to 3 for the production of a pharmaceutical for the prevention of tumor metastasis.

7. The use of a compound of the formula I or II as claimed in one or more of claims 1 to 3 for the production of a pharmaceutical for the treatment of inflammatory disorders.

## Revendications

1. Composé de Formule I 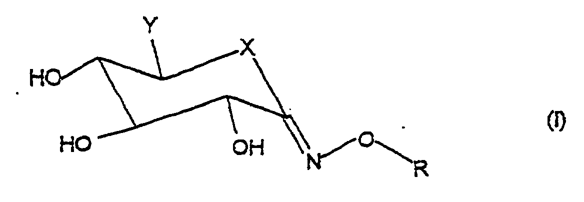 et/ou sel compatible d'un point de vue physiologique du composé de Formule I, où
Y est -COOH, -PO₃H₂, -P(O) (OR⁶) (OH), -P (O) R⁶ (OH), le groupe tétrazolyle ou -SO₃H, où R⁶ est un groupe alkyle en C₁ à C₄,
X est NH, O ou S,
R est un atome d'hydrogène ou -C(O)NHC₆(R⁷)₅, où les radicaux R⁷ représentent chacun indépendamment des autres un atome d'hydrogène, OH, un atome d'halogène, -COOH, -PO₃H₂ ou -SO₃H.

2. Composé de Formule II selon la revendication 1 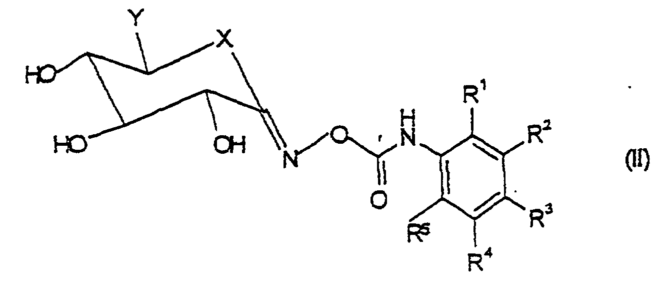 et/ou sel de sodium compatible d'un point de vue physiologique du composé de Formule II, où
Y est -COOH, -PO₃H₂, -P(O)(OR⁶)(OH), -P(O)R⁶(OH), où
R⁶ est un groupe alkyle en C₁ à C₄,
X est NH ou O,
R¹, R², R³, R⁴ et R⁵ représentent chacun indépendamment des autres un atome d'hydrogène, OH, un atome d'halogène, -COOH, -PO₃H₂ ou -SO₃H.

3. Composé de Formule II selon la revendication 1 ou 2, dans lequel
Y est -COOH ou -PO₃H₂,
X est NH ou O, et
R¹, R², R³, R⁴ et R⁵ représentent chacun indépendamment des autres un atome d'hydrogène ou de chlore.

4. Médicament contenant au moins un composé de Formule I ou II selon l'une ou plusieurs des revendications 1 à 3.

5. Utilisation d'un composé de Formule I ou II selon l'une ou plusieurs des revendications 1 à 3 pour préparer un médicament destiné au traitement de maladies de type cancer.

6. Utilisation d'un composé de Formule I ou II selon l'une ou plusieurs des revendications 1 à 3 pour préparer un médicament destiné à la prévention des métastases tumorales.

7. Utilisation d'un composé de Formule I ou II selon l'une ou plusieurs des revendications 1 à 3 pour préparer un médicament destiné au traitement de maladies inflammatoires.
